# EUROPEAN PATENT SPECIFICATION

(11) **EP 2 937 076 B1**
(45) Date of publication and mention of the grant of the patent: **11.09.2019**
(21) Application number: 15164494.5
(22) Date of filing: 21.04.2015
(51) Int. Cl.: A61K 9/00, A61K 47/02, A61K 47/10, A61K 47/14, A61K 31/5377, A61K 31/5575

(54) **EYE DROPS CONTAINING PROSTAGLANDIN AND TYLOXAPOL**
AUGENTROPFEN MIT PROSTAGLANDIN UND TYLOXAPOL
COLLYRE CONTENANT DE LA PROSTAGLANDINE ET DU TYLOXAPOL

(30) Priority: 25.04.2014 EP 14166033
(43) Date of publication of application: 28.10.2015
(73) Proprietor: Alfred E. Tiefenbacher (GmbH & Co. KG), 22767 Hamburg (DE); S.C. Rompharm Company S.R.L., 075100 Otopeni (RO)
(72) Inventor: Flemming, Jens, Dr., 22143 Hamburg (DE); Leanca, Rada, 042075 Bucarest (RO); Bocan, Andrei, Ploiesti (RO)
(74) Representative: Hamm&Wittkopp Patentanwälte PartmbB

(56) References cited:
- EP-A1- 2 077 105
- EP-A1- 2 478 906
- WO-A2-2006/050836
- WO-A2-2009/117316

## Description

The present invention relates to an aqueous ophthalmic solution comprising a prostaglandin F2α analogue and tyloxapol.

Prostaglandin F2α analogues have been known to be useful as ophthalmic pharmaceutical agents, specifically as ocular hypertensive antiglaucoma agents. For example, travoprost, latanoprost, tafluprost, bimatoprost and unoprostone isopropyl are marketed under the trade names Travatan®, Izba®, Xalatan®, Taflotan®, Lumigan® and, respectively, Rescula® as eye drop solutions for the treatment of ocular hypertension and glaucoma. These drugs lower intraocular pressure (IOP) by increasing outflow of aqueous humor through both the trabecular meshwork and uveoscleral routes, and they are thus applied to the eye for a localized action in the interior of the eye.

Prostaglandin F2α analogues are often administered with β-blockers and/or α2 agonists such as brimonidine (Alphagan®, Alphagan® P) to increase their IOP-lowering efficacy. For example, fixed ophthalmic combinations of travoprost, latanoprost, and respectively, bimatoprost with the β-blocker timolol are marketed under the trade names DuoTrav®, Xalacom® and Ganfort®.

A major problem in ocular therapeutics is the attainment of an optimal drug concentration at the site of action. The generally poor bioavailability of drugs from eye drops is mainly due to the pre-corneal loss caused by tear dynamics, systemic absorption, transient residence time in ocular surface, binding by the lacrimal proteins, limited corneal area, and the relative impermeability of the corneal epithelial membrane. Due to this physiological and anatomical constrains, only a small fraction of the administered drug is ocularly absorbed. This is particularly true for the prostaglandin F2α analogues which have poor water solubility.

Various formulation strategies have been used to increase aqueous solubility of these drugs and pre-ocular retention of eye drops. Different dosage forms have been suggested including micellar systems, emulsions, liposomes, and nanosized suspensions, which generally require the presence of surfactants in order to overcome insufficient drug solubility and increase efficiency of drug delivery. A particularly useful class of surfactants for applications in topical ocular drug delivery are polyoxyethylated nonionic surfactants (Advanced Drug Delivery Reviews 2008, 60, 1663-1673), e.g. polysorbates, cremophors, poloxamers and tyloxapol.

US 8,414,904 discloses an oil-in-water emulsion comprising a prostaglandin and at least two nonionic surfactants. The emulsion is preferably a microemulsion with oil droplets having an average size of less than 1 µm. Examples of suitable oil phase components include ethyl oleate, Miglyol® 812 (a mixture of C₈₋₁₀ fatty acid triglycerids), ricinus oil and corn oil, while the nonionic surfactants are preferably chosen from different polyoxyethylene sorbitan fatty acid esters, i.e. polysorbates (e.g. Tween® 20 or Tween® 80), and different polyoxyethylene cetylethers (e.g. Brij®52, Brij®56 and Brij®58). These oils and surfactants are commonly used in the preparation of microemulsions (ISRN Pharmaceutics 2013, article ID 826798).

EP 2 077 105 relates to an aqueous ophthalmic solution comprising a plurality of micelles having a size of 1 nm to 100 nm. The solution contains a prostaglandin, preferably latanoprost, which is incorporated into the core of the micelles. The micelles are composed of a mixture of n-alkyl dimethyl benzyl ammonium chlorides, whereby more than 30 % of the molecules have an alkyl chain length of ≥ C16. Compared to benzalkonium chloride, which is a commonly used preservative in ophthalmic formulations, the proportion of the quaternary ammonium compounds having an alkyl chain length of ≥ C16 is larger. Like benzalkonium chloride, the mixture of quaternary ammonium compounds disclosed in EP 2 077 105 forms cationic micelles. However, the described mixture of quaternary ammonium compounds is more lipophilic than benzalkonium chloride, which allows the use of lower concentrations of the cationic surfactant, which in turn minimizes the toxicity. It is commonly known that benzalkonium chloride is cytotoxic to both corneal and conjunctival cells (Adv. Ther. 2010, 27(11), 837-845). The micellar aqueous solution may contain nonionic surfactants, such as tyloxapol and cremophor, in order to enhance the affinity of the cationic micelles for the corneal surface.

EP 1 321 144 pertains to an ophthalmic solution comprising a prostaglandin F2α analogue and a nonionic surfactant. The nonionic surfactant, preferably polysorbate 80 or polyoxyethylene hydrogenated castor oil 60, should prevent the adsorption of the prostaglandin analogue to the inert surface of a container made of a resin, e.g. polyethylene or polypropylene.

WO 2009/117242 pertains to an aqueous ophthalmic composition, preferably a solution, containing a drug, preferably travoprost, a first polyol selected from mannitol and sorbitol, a second polyol selected from propylene glycol and glycerin, boric acid and a preservative, preferably a polymeric quaternary ammonium compound, e.g. polyquaternium-1. According to WO 2009/117242, polyols and boric acid form a borate-polyol complex. It was found that the borate-polyol complex produces superior antimicrobial activity, while the borate's buffering efficacy is maintained. Preferably, the aqueous ophthalmic solution contains a nonionic surfactant, e.g. polyoxyethylene hydrogenated castor oils, and no benzalkonium chloride.

WO 2009/117316 relates to an aqueous ophthalmic solution containing a prostaglandin, preferably travoprost, a polymeric quaternary ammonium compound as preservative, preferably polyquaternium-1, and a relatively low concentration of an ethoxylated and/or hydrogenated vegetable oil surfactant, preferably polyoxyethylene (40) hydrogenated castor oil. It was found that the bioavailability of the prostaglandin analogue is increased if the ophthalmic formulation only contains a low concentration of the surfactant. WO 2009/117316 also discloses an aqueous suspension containing brinzolamide and travoprost. The suspension contains a relatively high amount of polyoxyethylene (40) hydrogenated castor oil and a low amount of tyloxapol as an additional surfactant. It was found that the bioavailability of travoprost in this suspension is lower than in a corresponding suspension, which contains polyoxyethylene (40) hydrogenated castor oil in lower amounts and no tyloxapol. On the other hand, the concentration of the surfactant had nearly no effect on brinzolamide's bioavailability.

It was an objective of the present invention to provide a well tolerated aqueous ophthalmic solution containing a prostaglandin F2α analogue with improved bioavailability of the drug.

This objective is attained by the subject matter as defined in the claims.

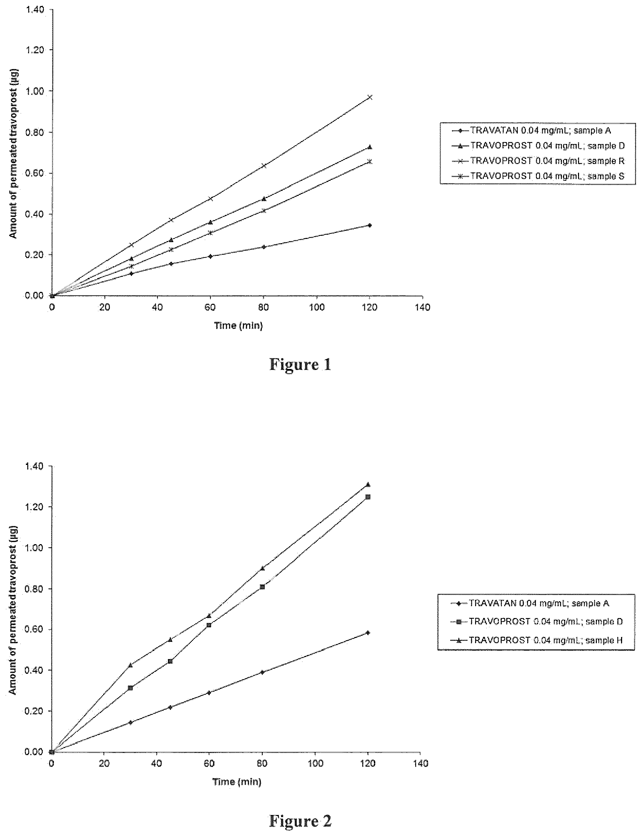

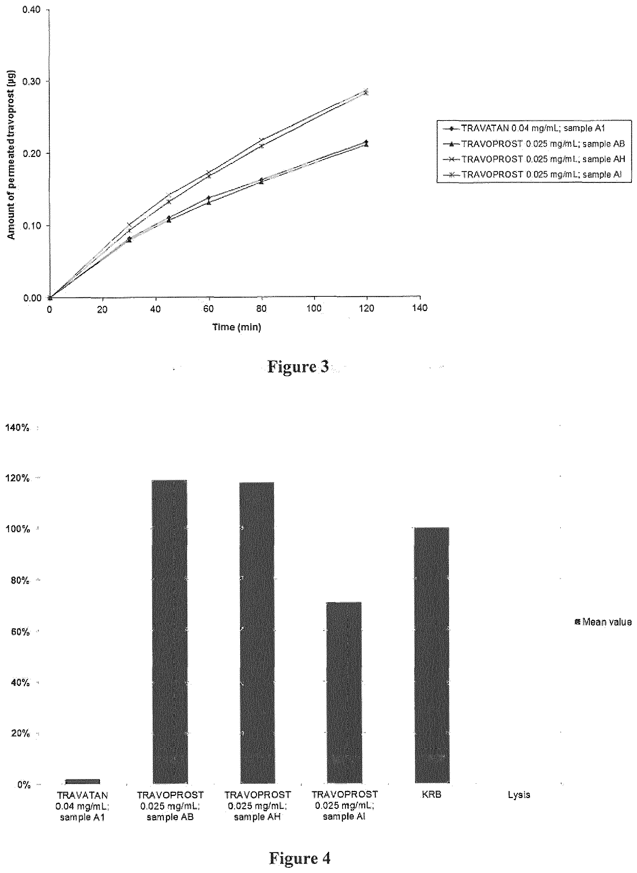

The present invention relates to an aqueous ophthalmic solution comprising a prostaglandin F2α analogue, tyloxapol as the only surfactant, a buffering agent and a cosolvent, wherein the prostaglandin F2a analogue is selected from travoprost, latanoprost, tafluprost and bimatoprost. It has been found that tyloxapol, which is a nonionic liquid polymer of the alkyl aryl polyether alcohol type, has a beneficial effect on the bioavailability of prostaglandin F2α analogues if it is the only surfactant in the aqueous solution. For example, the travoprost containing ophthalmic solution marketed under the trade name Travatan® contains Cremophor® (synonymous with Kolliphor®) RH-40 as nonionic surfactant. It could be established by in-vitro permeation studies that the substitution of this surfactant by tyloxapol increased the permeation of travoprost through the human and porcine cornea (approximately twice the amount of travoprost permeated through the cornea during the first two hours; Figures 1 to 3) and that this effect is not caused by injury of the cornea, as demonstrated in a cell proliferation and viability test (Figure 4). It has also been observed that the average size of the tyloxapol micelles formed in the aqueous solution as determined by photon correlation spectroscopy is much smaller than the micelles formed by cremophor. Consequently, the ophthalmic solution of the present invention does not contain any other surfactant than tyloxapol, neither the surfactants commonly employed in ophthalmic preparations for this purpose nor surfactants, which are contained in such solutions usually for other purposes, e.g. the preservative benzalkonium chloride. Examples of the buffering agent include boric acid, citric acid, acetic acid, phosphoric acid and trometamol, which form a buffer or a buffering complex with its conjugate base or acid. The buffer is usually formed when adjusting the pH of the aqueous ophthalmic solution to 5.5-7.5, preferably 6.5-7.0, with sodium hydroxide or hydrochloric acid. For the formation of micelles, cosolvents are usually added to the ophthalmic solution. Commonly employed are C₂₋₄-alkanols (i.e. 1,2/1,3/or 1,4-alkanediols), C₃₋₆-alkane polyols and polyethylene glycols of low molecular weight. Examples of suitable cosolvents include propylene glycol, 1,4-butanediol, glycerol and polyethylene glycols of low molecular weight.

The aqueous ophthalmic solution according to the present invention may contain a preservative and a tonicity adjusting agent. Examples of the preservative include parabens (methyl-, ethyl-, propyl-), zinc salts, preferably zinc chloride, and polyquaternium-1, whereby polyquaternium-1 is the most preferred preservative used in the present invention. Examples of the tonicity adjusting agent include sodium chloride, potassium chloride, mannitol and sorbitol.

The aqueous ophthalmic solution according to the present invention typically contains the prostaglandin F2α analogue, tyloxapol, the buffering agent and the cosolvents in the following concentrations:
0.0001-0.1 % (w/v) of the prostaglandin F2α analogue,
0.01-0.2 % (w/v) of tyloxapol,
0.01-1.0 % (w/v) of the buffering agent, and
0.25-5.0 % (w/v) of the cosolvent.

According to a preferred embodiment, the concentrations of the aforementioned components in the aqueous ophthalmic solution are:
0.001-0.05 % (w/v) of the prostaglandin F2α analogue,
0.025-0.1 % (w/v) of tyloxapol,
0.1-1.0 % (w/v) of the buffering agent, and
0.5-2.0 % (w/v) of the cosolvent.

If contained in the ophthalmic solution of the present invention, the concentration of the preservative is 0.0005-0.05 % (w/v), preferably 0.001-0.02 % (w/v), and if a tonicity adjusting agent is contained in the solution, it is present in a concentration of 0.2-6.0 % (w/v), preferably 0.3-5.0 % (w/v).

Preferably, the aqueous ophthalmic solution according to the present invention contains travoprost. Accordingly, the present invention relates to an aqueous ophthalmic solution containing travoprost, the components of which are present in the concentrations:
0.002-0.004 % (w/v) of travoprost,
0.05-0.075 % (w/v) of tyloxapol,
0.01-0.5 % (w/v) of the buffering agent,
0.5-2.0 % (w/v) of the cosolvent, and if contained,
0.001-0.002 % (w/v) of the preservative and
0.3-5.0 % (w/v) of the tonicity adjusting agent.

Preferably, boric acid or a combination of citric acid and trometamol is used for buffering the travoprost containing ophthalmic solution. This solution preferably contains propylene glycol or mannitol, and more preferred a combination of these sugar alcohols. The travoprost containing ophthalmic solution may contain polyquaternium-1 as preservative and sodium chloride as tonicity adjusting agent.

The ophthalmic solution according to the present invention may comprise an additional active ingredient, e.g. diclofenac sodium, bromfenac sodium or timolol maleate, each preferably in combination with travoprost.

### Examples

*In-vitro* permeation study

### Test model 3D HC model

### Test model:

For the in-vitro permeation study a standardized, three-dimensional (3D) model of the human cornea (Hemicornea, HC) was used. The model is described in detail by Hahne et al. in J. Pharm. Sci. 2012, 101(8), 2976-2988.

The serum-free cultivated cornea equivalent (HC) model is based on immortalized HCE-T cells and immortalized human corneal keratocytes (HCK) that are embedded in a collagenous stromal matrix.

### Cultivation of cell lines:

The cultivation and procedure followed in general the approach described by Hahne et al. in J. Pharm. Sci. 2012, 101(8), 2976-2988.

Both cell lines were cultivated according to established SOP (Hahne et al. in Int. J. Pharm. 2011, 416(1), 268-279) in 25 cm² tissue culture flask at 37 °C in a humidified atmosphere containing 5 % CO₂ using a serum-free growth medium (KGM). This medium contained 30 µg/mL bovine pituitary extract, 0.1 ng/mL human epidermal growth factor, 0.5 µg/mL hydrocortisone, 5 µg/mL insulin, 0.5 mM calcium chloride, 15 ng/mL amphotericin B, and 30 µg/mL gentamycin sulfate.

The growth medium was replaced three times per week, and the cultures were tested regularly for the absence of mycoplasma infections using the MycoTrace polymerase chain reaction detection kit. Cells were subculture when they reached confluence, at which time the medium was removed and the culture washed with PBS.

Subsequently, HCE-T cells were incubated for 2 min in EDTA (2 %) and for 8 min in trypsin-EDTA (0.05 % - 0.022 %) to detach them from the flask. In contrast, HCK cells were detached by incubating them in trypsin-EDT for 4 min. The trypsinization of both cultures was stopped by adding soybean trypsin inhibitor. The cells were pelleted by centrifugation and used for HC construction (or seeded into new culture flask).

### Cultivation of HC model:

Cultivation of the 3D HC was performed in 12-well Transwell® inserts as per SOP. To form the stromal equivalent, HCK cells suspended in a solution of 10 x minimum essential medium (MEM), L-glutamine (12.9 mM), and NaHCO₃ (16.1 mg/mL) were mixed with a solution of type 1 rat tail collagen in acetic acid (1.7 mg/mL) and cast into the Transwell® (polycarbonate, 3.0 µm pore size). This collagen matrix contained 80,000 HCK cells per Transwell® and gelled within 1 h. Subsequently, 1.5 mL KGM culture medium was added under the filter, and a suspension of 100,000 HCE-T cells in culture medium were seeded onto each stromal equivalent. The HC was first cultivated, submerged in medium for 7 days, and then cultivated at the ALI for 3 more days. The culture medium was replaced every other day during the submerged cultivation and once daily after the HC was transferred to the ALI. The permeation analysis was performed normally on day 9 of cultivation.

The barrier function of the HC was evaluated by determining the trans-epithelial electrical resistance (TEER) value reflecting mainly the resistance across epithelial tight junctions.

### Testing procedure:

The formulations (test batches) were used as is. However, for quantification of travoprost ³H-labelled travoprost was given to each formulation (donor concentration 1 µCi/mL).

The permeation studies with the HC were performed directly in the Transwell® at 37 °C. The HC was rinsed with KRB and incubated for 30 min with tempered KRB, which was removed before the formulation was applied to the epithelial side. During the exposure (application) time of 5 min and 10 min, respectively, for each test formulation no acceptor was present. Afterwards the formulation was completely removed from the epithelial layer and the donor side was rinsed two times with KBR. By transferring the Transwell® inserts to the acceptor compartment the permeation started. After 30, 45, 60, 80, and 120 min samples of 200 µL were taken from the acceptor compartment and replaced by the same volume of tempered KRB. Acceptor solutions were agitated continuously using an orbital shaker throughout the permeation.

The quantification of ³H-labelled travoprost was done using a liquid scintillation counter (LS6500, Beckman Coulter) following the addition of each sample to scintillation liquid (OptiPhase SuperMix).

### Test model Porcine cornea

### Test model:

Porcine corneas.

### Testing procedure:

See Hahne et al. in J. Pharm. Sci. 2012, 101(8), 2976-2988.

Porcine corneas were obtained from a local slaughterhouse and from local breeders. Animals were bred and slaughtered for human consumption. All corneas were removed within 30 min of death and transported in KRB to assure the preservation of their physiological properties.

For permeation testing the excised corneas were treated in an analogous manner, as reported for the 3D HC model. However, formulation's exposure time was 10 min. The permeation study was performed using a vertical Using diffusion chamber system (Harvard Apparatus, Holliston, Massachusetts).

The quantification of ³H-labelled travoprost was done using a liquid scintillation counter (LS6500, Beckman Coulter) following the addition of each sample to scintillation liquid (OptiPhase SuperMix).

### Test model cell proliferation assay (cell viability test)

### Test model:

HCE-T cells.

### Testing procedure:

The general procedure followed the approach described by Huhtala et al. in J. Ocul. Pharmacol. Ther. 2003, 19(1), 11-21.

The study was performed to investigate the influence of formulations on cell proliferation and cell viability. Both are indicative parameters for formulations' safety and of tolerability. Immortalized HCE-T cells were cultivated following internal procedures. The cells were exposed to formulations for 2 x 10 min for 4 days and for 10 min at day 5. After that the viability of cells were determined by MTS assay (see Malich et al. in Toxicology 1997, 124(3), 179-192). For both negative and positive control of test model the cells were treated with Krebs-Ringer and lysis buffer medium as well.

### Example 1

| **Sample No.** | **Batch No.** | **Formulation** | **Content travoprost** | **Composition** | |
|---|---|---|---|---|---|
| A | 11K07A | TRAVATAN® eye drops, solution; Alcon Laboratories (UK) Ltd.; sample ex DE | 0.04 mg/mL | Travoprost | 0.04 mg/mL |
| | | | | Polyquaternium-1 | 0.01 mg/mL |
| | | | | Polyoxyethylene hydrogenated castor oil 40 (i.e. HCO-40; macrogolglycerol hydroxystearate) | 2 mg/mL |
| | | | | Boric acid | N/S |
| | | | | Mannitol | N/S |
| | | | | Sodium chloride | N/S |
| | | | | Propylene glycol | 7.5 mg/mL |
| | | | | Sodium hydroxide and/or hydrochloric acid (to adjust pH) | 6.7 - 6.9 (as per analytical result) |
| | | | | Purified water | ad 1 mL |
| | | | | *Note: Reference product by Alcon; quantitative* / *qualitative composition as per TRAVATAN*® *SmPC (dated 07*/*2014)* | |
| A1 | 13F18J | TRAVATAN® eye drops, solution; Alcon Laboratories (UK) Ltd.; sample ex DE | 0.04 mg/mL | See above | |
| D | 320 | TRAVOPROST eye drops, solution | 0.04 mg/mL | Travoprost | 0.04 mg/mL |
| | | | | Polyquaternium-1 | 0.01 mg/mL |
| | | | | Tyloxapol | 0.5 mg/mL |
| | | | | Boric acid | 3 mg/mL |
| | | | | Mannitol | 8.5 mg/mL |
| | | | | Sodium chloride | 3.4 mg/mL |
| | | | | Propylene glycol | 7.5 mg/mL |
| | | | | Sodium hydroxide and/or | qs ad |
| | | | | hydrochloric acid (1 M) | pH 6.8 ± 0.1 |
| | | | | Purified water | ad 1 mL |
| H | 0412 | TRA VOPROST eye drops, solution; preservative free | 0.04 mg/mL | Travoprost | 0.04 mg/mL |
| | | | | Tyloxapol | 0.5 mg/mL |
| | | | | Boric acid | 3 mg/mL |
| | | | | Mannitol | 8.5 mg/mL |
| | | | | Sodium chloride | 3.4 mg/mL |
| | | | | Propylene glycol | 7.5 mg/mL |
| | | | | Sodium hydroxide and/or | qs ad |
| | | | | hydrochloric acid (1 M) | pH 6.8 ± 0.1 |
| | | | | Purified water | ad 1 mL |
| R | 357 | TRAVOPROST eye drops, solution | 0.04 mg/mL | Travoprost | 0.04 mg/mL |
| | | | | Polyquaternium-1 | 0.01 mg/mL |
| | | | | Tyloxapol | 0.2 mg/mL |
| | | | | Boric acid | 3 mg/mL |
| | | | | Mannitol | 8.5 mg/mL |
| | | | | Sodium chloride | 3.4 mg/mL |
| | | | | Propylene glycol | 7.5 mg/mL |
| | | | | Sodium hydroxide and/or | qs ad |
| | | | | hydrochloric acid (1 M) | pH 6.8 ± 0.1 |
| | | | | Purified water | ad 1 mL |
| S | TY0075 | TRAVOPROST eye drops, solution | 0.04 mg/mL | Travoprost | 0.04 mg/mL |
| | | | | Polyquaternium-1 | 0.01 mg/mL |
| | | | | Tyloxapol | 0.75 mg/mL |
| | | | | Boric acid | 3 mg/mL |
| | | | | Mannitol | 8.5 mg/mL |
| | | | | Sodium chloride | 3.4 mg/mL |
| | | | | Propylene glycol | 7.5 mg/mL |
| | | | | Sodium hydroxide and/or | qs ad |
| | | | | hydrochloric acid (1 M) | pH 6.8 ± 0.1 |
| | | | | Purified water | ad 1 mL |
| AB | 119 | TRA VOPROST eye drops, solution; preservative free | 0.025 mg/mL | Travoprost | 0.025 mg/mL |
| | | | | Tyloxapol | 0.5 mg/mL |
| | | | | Boric acid | 3 mg/mL |
| | | | | Mannitol | 8.5 mg/mL |
| | | | | Sodium chloride | 3.4 mg/mL |
| | | | | Propylene glycol | 7.5 mg/mL |
| | | | | Sodium hydroxide and/or | qs ad |
| | | | | hydrochloric acid (1 M) | pH 6.8 ± 0.1 |
| | | | | Purified water | ad 1 mL |
| AH | 126 | TRAVOPROST eye drops, solution; preservative free | 0.025 mg/mL | Travoprost | 0.025 mg/mL |
| | | | | Tyloxapol | 0.5 mg/mL |
| | | | | Trometamol | 1.2 mg/mL |
| | | | | Citric acid, monohydrate | 0.675 mg/mL |
| | | | | Mannitol | 46 mg/mL |
| | | | | Sodium hydroxide and/or | qs ad |
| | | | | hydrochloric acid (1 M) | pH 6.8 ± 0.1 |
| | | | | Purified water | ad 1 mL |
| Al | 127 | TRAVOPROST eye drops, solution; preservative free | 0.025 mg/mL | Travoprost | 0.025 mg/mL |
| | | | | Tyloxapol | 0.5 mg/mL |
| | | | | Trometamol | 1.2 mg/mL |
| | | | | Citric acid, monohydrate | 0.675 mg/mL |
| | | | | Glycerol | 25 mg/mL |
| | | | | Sodium hydroxide and/or | qs ad |
| | | | | hydrochloric acid (1 M) | pH 6.8 ± 0.1 |
| | | | | Purified water | ad 1 mL |

### Study results 3D HC model; exposure time 10 min

The *in-vitro* permeation study results are as follows:

| | **TRAVATAN® 0.04 mg/mL eye drops, solution; batch No. 11K07A (sample A)** | | **TRAVOPROST 0.04 mg/mL eye drops, solution; batch No. 320 (sample D)** | | **TRAVOPROST 0.04 mg/mL eye drops, solution; batch No. 357 (sample R)** | | **TRAVOPROST 0.04 mg/mL eye drops, solution; batch No. TY0075 (sample S)** | |
|---|---|---|---|---|---|---|---|---|
| **Sampling time** | **Amount of permeated travoprost (µg)** | | | | | | | |
| | **Mean** | **SD** | **Mean** | **SD** | **Mean** | **SD** | **Mean** | **SD** |
| 0 min | 0.00E+00 | 0.00E+00 | 0.00E+00 | 0.00E+00 | 0.00E+00 | 0.00E+00 | 0.00E+00 | 0.00E+00 |
| 30 min | 1.06E-01 | 1.13E-02 | 1.81E-01 | 4.30E-02 | 2.48E-01 | 1.35E-01 | 1.43E-01 | 5.45E-02 |
| 45 min | 1.57E-01 | 1.84E-02 | 2.74E-01 | 5.31E-02 | 3.72E-01 | 1.99E-01 | 2.24E-01 | 7.39E-02 |
| 60 min | 1.92E-01 | 2.32E-02 | 3.63E-01 | 6.31E-02 | 4.78E-01 | 2.34E-01 | 3.07E-01 | 1.21E-01 |
| 80 min | 2.39E-01 | 3.02E-02 | 4.77E-01 | 9.00E-02 | 6.36E-01 | 3.13E-01 | 4.19E-01 | 1.83E-01 |
| 120 min | 3.46E-01 | 5.18E-02 | 7.30E-01 | 9.26E-02 | 9.72E-01 | 4.03E-01 | 6.59E-01 | 2.74E-01 |

| | **TRAVOPROST 0.04 mg/mL eye drops, solution; batch No. 320 (sample D)** | | | **TRAVOPROST 0.04 mg/mL eye drops, solution; batch No. 357 (sample R)** | | **TRAVOPROST 0.04 mg/mL eye drops, solution; batch No. TY0075 (sample S)** | | |
|---|---|---|---|---|---|---|---|---|
| | **Ratio** | | | | | | | |
| | **permeated amount travoprost** | | | | | | | |
| **Sampling time** | **TRAVATAN® 0.04 mg/mL eye drops, solution; batch No. 11K07A (sample A)** | | | | | | | |
| | **related to permeated amount travoprost sample x** | | | | | | | |
| 0 min | N/A | | | N/A | | N/A | | |
| 30 min | 1 : 1.70 | | | 1 : 2.34 | | 1 : 1.35 | | |
| 45 min | 1 : 1.75 | | | 1 : 2.38 | | 1 : 1.43 | | |
| 60 min | 1 : 1.89 | | | 1 : 2.48 | | 1 : 1.60 | | |
| 80 min | 1 : 2.00 | | | 1 : 2.67 | | 1 : 1.75 | | |
| 120 min | 1 : 2.11 | | | 1 : 2.81 | | 1 : 1.91 | | |

### Study results Porcine cornea; exposure time 10 min

The *in-vitro* permeation study results are as follows:

| | **TRAVATAN® 0.04 mg/mL eye drops, solution; batch No. 11K07A (sample A)** | | **TRAVOPROST 0.04 mg/mL eye drops, solution; batch No. 320 (sample D)** | | **TRAVOPROST 0.04 mg/mL eye drops, solution; batch No. 0412 (sample H)** | |
|---|---|---|---|---|---|---|
| **Sampling time** | **Amount of permeated travoprost (µg)** | | | | | |
| | **Mean** | **SD** | **Mean** | **SD** | **Mean** | **SD** |
| 0 min | 0.00E+00 | 0.00E+00 | 0.00E+00 | 0.00E+00 | 0.00E+00 | 0.00E+00 |
| 30 min | 1.44E-01 | 5.13E-02 | 3.14E-01 | 1.78E-01 | 4.27E-01 | 1.16E-01 |
| 45 min | 2.17E-01 | 5.27E-02 | 4.44E-01 | 2.17E-01 | 5.51E-01 | 1.86E-01 |
| 60 min | 2.90E-01 | 4.72E-02 | 6.22E-01 | 2.76E-01 | 6.69E-01 | 2.14E-01 |
| 80 min | 3.89E-01 | 6.62E-02 | 8.10E-01 | 3.07E-01 | 9.02E-01 | 3.12E-01 |
| 120 min | 5.84E-01 | 8.56E-02 | 1.25E+00 | 3.20E-01 | 1.31E+00 | 3.58E-01 |

| | **TRAVOPROST 0.04 mg/mL eye drops, solution; batch No. 320 (sample D)** | | | **TRAVOPROST 0.04 mg/mL eye drops, solution; batch No. 0412 (sample H)** | | |
|---|---|---|---|---|---|---|
| | **Ratio** | | | | | |
| | **permeated amount travoprost** | | | | | |
| **Sampling time** | **TRAVATAN® 0.04 mg/mL eye drops, solution; batch No. 11K07A (sample A)** | | | | | |
| | **related to permeated amount travoprost sample x** | | | | | |
| 0 min | N/A | | | N/A | | |
| 30 min | 1 : 2.18 | | | 1 : 2.96 | | |
| 45 min | 1 : 2.04 | | | 1 : 2.53 | | |
| 60 min | 1 : 2.15 | | | 1 : 2.31 | | |
| 80 min | 1 : 2.08 | | | 1 : 2.32 | | |
| 120 min | 1 : 2.14 | | | 1 : 2.25 | | |

### Study results 3D HC model; exposure time 5 min

The *in-vitro* permeation study results are as follows:

| | **TRAVATAN® 0.04 mg/mL eye drops, solution; batch No. 13F18J (sample A1)** | | **TRAVOPROST 0.025 mg/mL eye drops, solution; batch No. 119 (sample AB)** | | **TRAVOPROST 0.025 mg/mL eye drops, solution; batch No. 126 (sample AH)** | | **TRAVOPROST 0.025 mg/mL eye drops, solution; batch No. 127 (sample AI)** | |
|---|---|---|---|---|---|---|---|---|
| **Sampling time** | **Amount of permeated travoprost (µg)** | | | | | | | |
| | **Mean** | **SD** | **Mean** | **SD** | **Mean** | **SD** | **Mean** | **SD** |
| 0 min | 0.00E+00 | 0.00E+00 | 0.00E+00 | 0.00E+00 | 0.00E+00 | 0.00E+00 | 0.00E+00 | 0.00E+00 |
| 30 min | 8.17E-02 | 2.93E-03 | 8.00E-02 | 3.72E-03 | 1.01E-01 | 1.30E-02 | 9.31E-02 | 1.32E-02 |
| 45 min | 1.10E-01 | 9.03E-03 | 1.07E-01 | 2.97E-03 | 1.42E-01 | 2.01E-02 | 1.32E-01 | 2.47E-02 |
| 60 min | 1.37E-01 | 1.23E-02 | 1.31E-01 | 1.80E-03 | 1.72E-01 | 2.88E-02 | 1.67E-01 | 2.80E-02 |
| 80 min | 1.62E-01 | 1.19E-02 | 1.59E-01 | 4.00E-03 | 2.17E-01 | 4.24E-02 | 2.09E-01 | 4.34E-02 |
| 120 min | 2.14E-01 | 1.91E-02 | 2.10E-01 | 4.17E-03 | 2.85E-01 | 4.79E-02 | 2.82E-01 | 6.04E-02 |

| | **TRAVOPROST 0.025 mg/mL eye drops, solution; batch No. 119 (sample AB)** | | | **TRAVOPROST 0.025 mg/mL eye drops, solution; batch No. 126 (sample AH)** | | **TRAVOPROST 0.025 mg/mL eye drops, solution; batch No. 127 (sample AI)** | | |
|---|---|---|---|---|---|---|---|---|
| | **Ratio** | | | | | | | |
| | **permeated amount travoprost** | | | | | | | |
| **Sampling time** | **TRAVATAN® 0.04 mg/mL eye drops, solution; batch No. 13F18J (sample A1)** | | | | | | | |
| | **related to permeated amount travoprost sample x** | | | | | | | |
| 0 min | N/A | | | N/A | | N/A | | |
| 30 min | 1 : 0.98 | | | 1 : 1.23 | | 1 : 1.14 | | |
| 45 min | 1 : 0.97 | | | 1 : 1.29 | | 1 : 1.20 | | |
| 60 min | 1 : 0.95 | | | 1 : 1.25 | | 1 : 1.21 | | |
| 80 min | 1 : 0.98 | | | 1 : 1.34 | | 1 : 1.29 | | |
| 120 min | 1 : 0.98 | | | 1 : 1.33 | | 1 : 1.32 | | |

### Study results cell proliferation assay (cell viability test)

The *in-vitro* cell viability study results are as follows:

| **Result** | **TRAVATAN® 0.04 mg/mL eye drops, solution; batch No. 13F18J (sample A1)** | **TRAVOPROST 0.025 mg/mL eye drops, solution; batch No. 119 (sample AB)** | **TRAVOPROST 0.025 mg/mL eye drops, solution; batch No. 126 (sample AH)** | **TRAVOPROST 0.025 mg/mL eye drops, solution; batch No. 127 (sample AI)** | **Krebs-Ringer buffer** | **Lysis buffer** |
|---|---|---|---|---|---|---|
| Mean cell viability after exposure | 2 % | 119 % | 118 % | 71 % | 100 % | 0 % |
| RSD | 9.3 % | 14.9 % | 14.7 % | 27.6 % | 10.9 % | 0 % |

Figure 1 shows the permeation profile for travoprost in the 3D HC model (exposure time 10 min, mean values).
Figure 2 shows the permeation profile for travoprost with porcine cornea (exposure time 10 min, mean values).
Figure 3 shows the permeation profile for travoprost in the 3D HC model (exposure time 5 min, mean values).
Figure 4 shows the results on cell proliferation assay (exposure time 2 x 10 min for 4 days, 1 x 10 min at day 5).

### Examples 2 to 5

### Manufacturing method (general procedure boric acid):

The starting materials (ingredients) are supplied and weighted according to the batch formula. Travoprost is solubilized as a stock solution in tyloxapol, propylene glycol and purified water by heating up to (45 ± 2) °C. Under stirring the solution is cooled to the environmental temperature.

Polyquaternium-1 is dissolved with purified water to obtain a polyquaternium-1 stock solution. Another beaker is charged with about 90 % of the total amount of purified water. The weighed quantities of boric acid, mannitol and sodium chloride are added under stirring into the beaker (preparation vessel) and stirred. The polyquaternium-1 (stock) solution is quantitatively added under stirring into the preparation vessel. The pH value of the solution is adjusted to 6.8 ± 0.1 by means of NaOH and/or HCl (1 M) solutions.

The travoprost stock solution is quantitatively added into the preparation vessel, the beaker of the travoprost stock solution is gradually rinsed with sufficient amount of purified water that is added into the preparation vessel, and finally the solution is stirred. The pH is checked and adjusted, if necessary. Purified water is added to reach the final volume. Sterile filtration of the bulk solution is carried out using a suitable filter capsule with a nominal pore size of 0.2 µm.

### Example 2

| g/100 mL | |
|---|---|
| Travoprost | 0.0020 |
| Boric acid | 0.300 |
| Sodium chloride | 0.340 |
| Mannitol | 0.850 |
| Tyloxapol | 0.050 |
| Poiyquaternium-1 | 0.001 |
| Propylene glycol | 0.750 |
| NaOH/HCl (1 M) | pH 6.7 - 6.9 |
| Purified water | ad 100 mL |

### Example 3

| g/100 mL | |
|---|---|
| Travoprost | 0.0025 |
| Boric acid | 0.300 |
| Sodium chloride | 0.340 |
| Mannitol | 0.850 |
| Tyloxapol | 0.050 |
| Polyquaternium-1 | 0.001 |
| Propylene glycol | 0.750 |
| NaOH/HCl (1 M) | pH 6.7 - 6.9 |
| Purified water | ad 100 mL |

### Example 4

| g/100 mL | |
|---|---|
| Travoprost | 0.0030 |
| Boric acid | 0.300 |
| Sodium chloride | 0.340 |
| Mannitol | 0.850 |
| Tyloxapol | 0.075 |
| Polyquaternium-1 | 0.001 |
| Propylene glycol | 0.750 |
| NaOH/HCl (1 M) | pH 6.7 - 6.9 |
| Purified water | ad 100 mL |

### Example 5

| g/100 mL | |
|---|---|
| Travoprost | 0.0040 |
| Boric acid | 0.300 |
| Sodium chloride | 0.340 |
| Mannitol | 0.850 |
| Tyloxapol | 0.100 |
| Polyquaternium-1 | 0.001 |
| Propylene glycol | 0.750 |
| NaOH/HCl (1 M) | pH 6.7 - 6.9 |
| Purified water | ad 100 mL |

### Example 6

### Manufacturing method (general procedure trometamol/citric acid):

The starting materials (ingredients) are supplied and weighted according to the batch formula. Travoprost is solubilized as a stock solution in tyloxapol, propylene glycol and purified water by heating up to (45 ± 2) °C. Under stirring the solution is cooled to the environmental temperature.

Another beaker is charged with about 90 % of the total amount of purified water. The weighed quantities of trometamol, citric acid and mannitol are added under stirring into the beaker (preparation vessel) and stirred for complete solubilization. The pH value of the solution is adjusted to 6.8 ± 0.1 by means of NaOH and/or HCl (1 M) solutions.

The travoprost stock solution is quantitatively added into the preparation vessel, the beaker of the travoprost stock solution is gradually rinsed with sufficient amount of purified water that is added into the preparation vessel, and finally the solution is stirred. The pH is checked and adjusted, if necessary. Purified water is added to reach the final volume. Sterile filtration of the bulk solution is carried out using a suitable filter capsule with a nominal pore size of 0.2 µm.

| g/1 00mL | |
|---|---|
| Travoprost | 0.0025 |
| Trometamol | 0.1200 |
| Citric acid x H₂O | 0.0675 |
| Mannitol | 4.600 |
| Tyloxapol | 0.050 |
| Propylene glycol | 0.750 |
| NaOH/HCl (1 M) | pH 6.7 - 6.9 |
| Purified water | ad 100 mL |

### Example 7

| g/100mL | |
|---|---|
| Travoprost | 0.0025 |
| Trometamol | 0.1200 |
| Citric acid x H₂O | 0.0675 |
| Mannitol | 4.600 |
| Tyloxapol | 0.050 |
| NaOH/HCl (1 M) | pH 6.7 - 6.9 |
| Purified water | ad 100 mL |

Departing from the general procedure (Example 6), a slurry of travoprost and tyloxapol without propylene glycol is initially formed. This mixture will be gradually diluted with heated water up to (40 - 45) °C, and stirring and heating will be ensured at least for 30 min (travoprost stock solution in tyloxapol will be 0.25 % (w/v)).

### Example 8

| g/100mL | |
|---|---|
| Travoprost | 0.0025 |
| Glycerol | 2.500 |
| Trometamol | 0.1200 |
| Citric acid x H₂O | 0.0675 |
| Tyloxapol | 0.050 |
| NaOH/HCl (1 M) | pH 6.7 - 6.9 |
| Purified water | ad 100 mL |

The travoprost solution will be prepared according to the general procedure (Example 6), except that propylene glycol is replaced by glycerol (only 30 % from theoretical quantity will be used for travoprost stock solution preparation, the remaining 70 % from glycerol will be added into vehicle).

### Example 9

| g/100 mL | |
|---|---|
| Travoprost | 0.0025 |
| Diclofenac sodium | 0.100 |
| Trometamol | 0.120 |
| Disodium edetate dihydrate | 0.050 |
| Mannitol | 4.000 |
| Tyloxapol | 0.050 |
| Propylene glycol | 0.750 |
| NaOH/HCl (1 M) | pH 7.2 ± 0.2 |
| Purified water | ad 100 mL |

The starting materials (ingredients) are supplied and weighted according to the batch formula. Travoprost is solubilized as a stock solution in tyloxapol, propylene glycol and purified water by heating up to (45 ± 2) °C. Under stirring the solution is cooled to the environmental temperature.

Another beaker is charged with about 90 % of the total amount of purified water. The weighed quantities of trometamol, disodium edetate and mannitol are added under stirring into the beaker (preparation vessel) and stirred for complete solubilization. The pH value of the solution is adjusted to 7.2 ± 0.2 by means of NaOH and/or HCl (1 M) solutions.

After adding travoprost stock solution into the preparation vessel, rinsing of beaker with water and stirring, diclofenac sodium is given into the preparation vessel. The pH is checked and adjusted, if necessary. Purified water is added to reach the final volume; the bulk solution is further stirred to ensure homogenous solution. Sterile filtration of the bulk solution is carried out using a suitable filter capsule with a nominal pore size of 0.2 µm.

### Example 10

| g/100 mL | |
|---|---|
| Travoprost | 0.0025 |
| Bromfenac sodium | 0.1035 |
| Trometamol | 0.1200 |
| Boric acid | 0.3000 |
| Disodium edetate dihydrate | 0.0200 |
| Mannitol | 3.8000 |
| Tyloxapol | 0.050 |
| Propylene glycol | 0.750 |
| NaOH/HCl (1 M) | pH 7.5 ± 0.2 |
| Purified water | ad 100 mL |

The starting materials (ingredients) are supplied and weighted according to the batch formula. Travoprost is solubilized as a stock solution in tyloxapol, propylene glycol and purified water by heating up to (45 ± 2) °C. Under stirring the solution is cooled to the environmental temperature.

Another beaker is charged with about 90 % of the total amount of purified water. The weighed quantities of trometamol, boric acid, disodium edetate and mannitol are added under stirring into the beaker (preparation vessel) and stirred for complete solubilization. The pH value of the solution is adjusted to 7.5 ± 0.2 by means of NaOH and/or HCl (1 M) solutions.

After adding travoprost stock solution into the preparation vessel, rinsing of beaker with water and stirring, bromfenac sodium is given into the preparation vessel. The pH is checked and adjusted, if necessary. Purified water is added to reach the final volume; the bulk solution is further stirred to ensure homogenous solution. Sterile filtration of the bulk solution is carried out using a suitable filter capsule with a nominal pore size of 0.2 µm.

### Example 11

| g/100 mL | |
|---|---|
| Travoprost | 0.0025 |
| Timolol maleate | 0.680 |
| Boric acid | 0.300 |
| Sodium chloride | 0.250 |
| Mannitol | 0.850 |
| Tyloxapol | 0.050 |
| Propylene glycol | 0.750 |
| NaOH/HCl (1 M) | pH 6.7 - 6.9 |
| Purified water | ad 100 mL |

The manufacture follows the routine given for Examples 2 to 5. However, after adding travoprost stock solution into the preparation vessel, rinsing of beaker with water and stirring, timolol maleate is given into the preparation vessel. The pH is checked and adjusted, if necessary. Purified water is added to reach the final volume; the bulk solution is further stirred to ensure homogenous solution. Sterile filtration of the bulk solution is carried out using a suitable filter capsule with a nominal pore size of 0.2 µm.

## Claims

1. An aqueous ophthalmic solution comprising a prostaglandin F2α analogue, tyloxapol as the only surfactant, a buffering agent and a cosolvent, wherein the prostaglandin F2α analogue is selected from travoprost, latanoprost, tafluprost and bimatoprost.

2. The aqueous ophthalmic solution according to claim 1, wherein the buffering agent is selected from boric acid, citric acid, acetic acid, phosphoric acid and trometamol, and is preferably boric acid or trometamol and citric acid.

3. The aqueous ophthalmic solution according to claim 1, wherein the cosolvent is selected from propylene glycol, glycerol and polyethylene glycols of low molecular weight.

4. The aqueous ophthalmic solution according to claims 1 to 3 comprising a preservative.

5. The aqueous ophthalmic solution according to claim 4, wherein the preservative is selected from parabens (methyl-, ethyl-, propyl-), zinc salts preferably zinc chloride, and polyquaternium-1, whereby polyquaternium-1 is preferred.

6. The aqueous ophthalmic solution according to claims 1 to 5 comprising a tonicity adjusting agent.

7. The aqueous ophthalmic solution according to claim 6, wherein the tonicity adjusting agent is selected from sodium chloride, potassium chloride, mannitol and sorbitol.

8. The aqueous ophthalmic solution according to claims 1 to 7, wherein the concentration of the:
- prostaglandin F2α analogue is 0.0001-0.1 % (w/v), preferably 0.001-0.05 % (w/v),
- surfactant tyloxapol is 0.01-0.2 % (w/v), preferably 0.025-0.1 % (w/v)
- buffering agent is 0.01-1.0 % (w/v), preferably 0.1-1.0 % (w/v)
- cosolvent is 0.25-5.0 % (w/v), preferably 0.5-2.0 % (w/v), and
wherein optionally the concentration of the:
- preservative is 0.0005-0.05 % (w/v), preferably 0.001 to 0.02 % (w/v)
- tonicity adjusting agent is 0.2-6.0 % (w/v), preferably 0.3-5.0 % (w/v).

9. The aqueous ophthalmic solution according to any one of the preceding claims, wherein the prostaglandin F2α analogue is travoprost.

10. The aqueous ophthalmic solution according to claim 9, wherein the concentration of the:
- prostaglandin F2α analogue travoprost is 0.002-0.004 % (w/v)
- surfactant tyloxapol is 0.05-0.075 % (w/v)
- buffering agent is 0.01-0.5 % (w/v)
- cosolvent is 0.5-2.0 % (w/v), and
wherein optionally the concentration of the:
- preservative is 0.001 to 0.002 % (w/v)
- tonicity adjusting agent is 0.3-5.0 % (w/v).

11. The aqueous ophthalmic solution according to claim 9, wherein the buffering agent is boric acid or a combination of citric acid and trometamol, the cosolvent is selected from propylene glycol and glycerol, preferably propylene glycol, the preservative is polyquaternium-1 and the tonicity adjusting agent is selected from sodium chloride and mannitol.

12. The aqueous ophthalmic solution according to any one of the preceding claims comprising an additional active ingredient.

13. The aqueous ophthalmic solution according to claim 12, wherein the additional active ingredient is selected from diclofenac sodium, bromfenac sodium and timolol maleate.

## Patentansprüche

1. Wässrige ophthalmische Lösung, enthaltend ein Prostaglandin F2α-Analogon, Tyloxapol als einzige grenzflächenaktive Substanz, einen Puffer und ein Co-Lösungsmittel, wobei das Prostaglandin F2α-Analogon aus Travoprost, Latanoprost, Tafluprost und Bimatoprost ausgewählt ist.

2. Wässrige ophthalmische Lösung gemäß Anspruch 1, wobei der Puffer aus Borsäure, Zitronensäure, Essigsäure, Phosphorsäure und Trometamol ausgewählt ist und bevorzugt Borsäure oder Trometamol und Zitronensäure ist.

3. Wässrige ophthalmische Lösung gemäß Anspruch 1, wobei das Co-Lösungsmittel aus Propylenglycol, Glycerin und Polyethylenglycolen mit geringem Molekulargewicht ausgewählt ist.

4. Wässrige ophthalmische Lösung gemäß den Ansprüchen 1-3, enthaltend ein Konservierungsmittel.

5. Wässrige ophthalmische Lösung gemäß Anspruch 4, wobei das Konservierungsmittel aus Parabenen (Methyl-, Ethyl-, Propyl-), Zinksalzen, vorzugsweise Zinkchlorid, und Polyquaternium-1 ausgewählt ist, wobei Polyquaternium-1 bevorzugt ist.

6. Wässrige ophthalmische Lösung gemäß den Ansprüchen 1-5, enthaltend ein Tonizität einstellendes Mittel.

7. Wässrige ophthalmische Lösung gemäß Anspruch 6, wobei das Tonizität einstellende Mittel aus Natriumchlorid, Kaliumchlorid, Mannitol und Sorbitol ausgewählt ist.

8. Wässrige ophthalmische Lösung gemäß Ansprüchen 1-7, wobei die Konzentration von:
- dem Prostaglandin F2α-Analogon 0.0001-0.1 % (w/v), bevorzugt 0,001-0,05 % (w/v) beträgt,
- der grenzflächenaktiven Substanz Tyloxapol 0,01-0,2 % (w/v), bevorzugt 0,025-0,1 % (w/v) beträgt,
- dem Puffer 0,01-1,0 % (w/v), bevorzugt 0,1-1,0 % (w/v),
- dem Co-Lösungsmittel 0,25-5,0 % (w/v), bevorzugt 0,5-2,0 % (w/v) beträgt, und
wobei gegebenenfalls die Konzentration von:
- dem Konservierungsmittel 0,0005-0,05 % (w/v), bevorzugt 0,001 bis 0.02 % (w/v) beträgt,
- dem Tonizität einstellenden Mittel 0.2-6.0 % (w/v), bevorzugt 0.3-5.0 % (w/v).

9. Wässrige ophthalmische Lösung gemäß einem der vorstehenden Ansprüche, wobei das Prostaglandin F2α-Analogon Travoprost ist.

10. Wässrige ophthalmische Lösung gemäß Anspruch 9, wobei die Konzentration von:
- dem Prostaglandin F2α-Analogon Travoprost 0,002-0,004 % (w/v) beträgt
- der grenzflächenaktiven Substanz Tyloxapol 0,05-0,075 % (w/v) beträgt,
- dem Puffer 0,01-0,5 % (w/v) beträgt,
- dem Co-Lösungsmittel 0,5-2,0 % (w/v) beträgt, und
wobei gegebenenfalls die Konzentration von:
- dem Konservierungsmittel 0,001 bis 0,002 % (w/v) beträgt,
- dem Tonizität einstellenden Mittel 0,3-5,0 % (w/v) beträgt.

11. Wässrige ophthalmische Lösung gemäß Anspruch 9, wobei der Puffer Borsäure oder eine Kombination von Zitronensäure und Trometamol ist, das Co-Lösungsmittel aus Propylenglycol und Glycerin ausgewählt ist, vorzugsweise Propylenglycol ist, das Konservierungsmittel Polyquaternium-1 ist und das Tonizität einstellende Mittel aus Natriumchlorid und Mannitol ausgewählt ist.

12. Wässrige ophthalmische Lösung gemäß einem der vorstehenden Ansprüche, enthaltend einen zusätzlichen Wirkstoff.

13. Wässrige ophthalmische Lösung gemäß Anspruch 12, wobei der zusätzliche Wirkstoff aus Diclofenac-Natrium, Bromfenac-Natrium und Timololmaleat ausgewählt ist.

## Revendications

1. Solution ophtalmique aqueuse comprenant un analogue de prostaglandine F2α, du tyloxapol comme seul tensioactif, un agent de tamponnage et un co-solvant, dans laquelle l'analogue de prostaglandine F2a est choisi parmi le travoprost, le latanoprost, le tafluprost et le bimatoprost.

2. Solution ophtalmique aqueuse selon la revendication 1, dans laquelle l'agent de tamponnage est choisi parmi l'acide borique, l'acide citrique, l'acide acétique, l'acide phosphorique et le trométamol, et est de préférence l'acide borique ou le trométamol et l'acide citrique.

3. Solution ophtalmique aqueuse selon la revendication 1, dans laquelle le co-solvant est choisi parmi le propylène glycol, le glycérol et les polyéthylène glycols de faible poids moléculaire.

4. Solution ophtalmique aqueuse selon les revendications 1 à 3, comprenant un agent de conservation.

5. Solution aqueuse ophtalmique selon la revendication 4, dans laquelle le conservateur est choisi parmi des parabènes (méthyl-, éthyl-, propyl-), des sels de zinc, de préférence le chlorure de zinc, et du polyquaternium-1, le polyquatemium-1 étant préféré.

6. Solution aqueuse ophtalmique selon les revendications 1 à 5, comprenant un agent d'ajustement de tonicité.

7. Solution aqueuse ophtalmique selon la revendication 6, dans laquelle l'agent d'ajustement de tonicité est choisi parmi le chlorure de sodium, le chlorure de potassium, le mannitol et le sorbitol.

8. Solution ophtalmique aqueuse selon les revendications 1 à 7, dans laquelle la concentration de :
- l'analogue de prostaglandine F2α est comprise entre 0,0001 et 0,1 % (poids/volume), de préférence entre 0,001 et 0,05 % (poids/volume),
- du tensioactif tyloxapol est comprise entre 0,01 et 0,2 % (poids/volume), de préférence entre 0,025 et 0,1 % (poids/volume)
- de l'agent de tamponnage est comprise entre 0,01 et 1,0 % (poids/volume), de préférence entre 0,1 et 1,0 % (poids/volume)
- du cosolvant est comprise entre 0,25 et 5,0% (poids/volume), de préférence entre 0,5 et 2,0 % (poids/volume), et
dans laquelle facultativement la concentration de :
- l'agent de conservation est compris entre 0,0005 et 0,05 % (poids/volume), de préférence entre 0,001 et 0,02 % (poids/volume)
- l'agent d'ajustement de tonicité est comprise entre 0,2 et 6,0 % (poids/volume), de préférence entre 0,3 et 5,0 % (poids/volume).

9. Solution aqueuse ophtalmique selon l'une quelconque des revendications précédentes, dans laquelle l'analogue de prostaglandine F2a est le travoprost.

10. Solution ophtalmique aqueuse selon la revendication 9, dans laquelle la concentration de :
- de travoprost analogue de prostaglandine F2a est comprise entre 0,002 et 0,004% (poids/volume)
- du tyloxapol tensioactif est comprise entre 0,05 et 0,075 % (poids/volume)
- de l'agent de tamponnage est comprise entre 0,01 et 0,5 % (poids/volume)
- du cosolvant est comprise entre 0,5 et 2,0 % (poids/volume), et
dans laquelle facultativement la concentration de :
- l'agent de conservation est comprise entre 0,001 et 0,002 % (poids/volume)
- l'agent d'ajustement de tonicité est comprise entre 0,3 et 5,0 % (poids/volume).

11. Solution ophtalmique aqueuse selon la revendication 9, dans laquelle l'agent de tamponnage est l'acide borique ou une combinaison d'acide citrique et de trométamol, le co-solvant est choisi parmi le propylène glycol et le glycérol, de préférence le propylène glycol, l'agent de conservation est le polyquatemium-1 et l'agent d'ajustement de tonicité est choisi parmi le chlorure de sodium et le mannitol.

12. Solution aqueuse ophtalmique selon l'une quelconque des revendications précédentes, comprenant un ingrédient actif supplémentaire.

13. Solution aqueuse ophtalmique selon la revendication 12, dans laquelle l'ingrédient actif supplémentaire est choisi parmi le diclofénac sodique, le bromfénac sodique et le maléate de timolol.
